(19) Europäisches Patentamt
European Patent Office
Office européen des brevets

(11) **EP 4 053 848 A1**

(12) **EUROPEAN PATENT APPLICATION**
published in accordance with Art. 153(4) EPC

(43) Date of publication:
**07.09.2022 Bulletin 2022/36**

(21) Application number: **20880663.8**

(22) Date of filing: **20.10.2020**

(51) International Patent Classification (IPC):
*G16H 20/30* (2018.01)    *G06Q 50/10* (2012.01)

(52) Cooperative Patent Classification (CPC):
**G06Q 50/10; G16H 20/30**

(86) International application number:
**PCT/JP2020/039364**

(87) International publication number:
**WO 2021/085233 (06.05.2021 Gazette 2021/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.11.2019  JP 2019199835**

(71) Applicant: **Astellas Pharma Inc.**
**Chuo-ku**
**Tokyo 103-8411 (JP)**

(72) Inventor: **KANAYAMA, Motohiro**
**Tokyo 103-8411 (JP)**

(74) Representative: **V.O.**
**P.O. Box 87930**
**2508 DH Den Haag (NL)**

(54) **EXERCISE SUPPORT DEVICE, EXERCISE SUPPORT SYSTEM, EXERCISE SUPPORT METHOD, AND PROGRAM**

(57)    An exercise support device (10) includes: an acquisition unit (11A) that acquires a physical quantity indicating an execution degree of an exercise according to an exercise program indicating a predetermined self-weight exercise; a deriving unit (11B) that derives subsequent intensity of the exercise by using the physical quantity acquired by the acquisition unit (11A); and a presentation unit (11C) that presents the intensity derived by the deriving unit (11B).

## FIG.2

**Description**

Technical Field

The technology of the present disclosure relates to an exercise support device, an exercise support system, an exercise support method, and a program.

Background Art

Conventionally, there have been the following techniques as techniques that can be applied to enable effective execution of exercise according to an exercise program.

Patent Document 1 discloses an exercise support device that appropriately evaluates a physical condition of a user during exercise. This exercise support device includes an evaluation means for evaluating a physical condition of a user from a combination of exercise data indicating an amount of exercise of the user and biological data indicating a physiological state of the user, and a determination means for determining an instruction content to be presented to the user by using an evaluation result obtained by the evaluation means. The evaluation means evaluates a physical condition of the user from a combination of a transition of the exercise data and a transition of the biological data.

Patent Document 2 discloses an exercise support system that provides exercise content corresponding to exercise ability of a user or a change in physical condition during exercise without hindering exercise of the user. This exercise support system includes: a display means that displays exercise content including a plurality of pieces of exercise information having different exercise intensities and displays exercise information to be provided to a user in a display format different from other exercise information; a biological data detection means that detects biological data of the user while the exercise content is displayed on the display means; a biological data determination means that determines whether or not the biological data detected by the biological data detection means is within a range of a predetermined target value of biological data in the exercise information to be provided to the user; and a display switching means that switches the exercise information to be provided to the user to other exercise information having different exercise intensities when the biological data determination means determines that the biological data is not within the range of the target value.

Patent Document 3 discloses a computer-implemented method for determining the physical condition of a subject for the purpose of strengthening the elderly and improving the quality of life and independence through an individualized lifestyle and nutrition program. The computer-implemented method includes steps of providing a classification system for nutritional and physical condition parameters of the subject, evaluating and scoring values of the parameters of the subject, classifying the subject into classes for each of the parameters using the scores obtained, and determining a physical condition of the subject using the classes determined for each parameter.

Citation List

Patent Document

Patent Document 1: Japanese Patent Application Laid-Open (JP-A) No. 2009-142333
Patent Document 2: Japanese Patent Application Laid-Open (JP-A) No. 2010-252985
Patent Document 3: Japanese National-Phase Publication (JP-A) No. 2016-507114

SUMMARY OF INVENTION

Technical Problem

In general, it is said that it is difficult to appropriately set the intensity of self-weight exercise in resistance exercise, that is, the exercise performed using own weight without using an apparatus (Japan Journal of Physical Education, Health and Sport Sciences, 57: 191-199, 2012).

For example, in a dumbbell exercise in the resistance exercise, intensity can be easily and appropriately set by adjusting the weight of a dumbbell. On the other hand, in a case of the self-weight exercise such as push-up (so-called push-ups), leg-raise, lunge, and self-weight squat, since there is no adjustment target capable of changing the intensity of the exercise, it is difficult to appropriately set the intensity of the exercise.

On the other hand, the techniques disclosed in the respective documents of Patent Documents 1 to 3 can contribute to the achievement of the object described in the corresponding document. However, since the setting of the intensity for the self-weight exercise is not taken into consideration, the intensity for the self-weight exercise cannot necessarily be appropriately set.

The technique of the present disclosure has been made in view of the above circumstances, and an object thereof is to provide an exercise support device, an exercise support system, an exercise support method, and a program capable of appropriately setting intensity against a self-weight exercise in the resistance exercise.

Solution to Problem

A first aspect of the present disclosure is an exercise support device including: an acquisition unit configured to acquire a physical quantity indicating an execution degree of an exercise according to an exercise program indicating a predetermined self-weight exercise; a deriving unit configured to derive subsequent intensity of the exercise by using the physical quantity acquired by the acquisition unit; and a presentation unit configured to present the intensity derived by the deriving unit.

A second aspect of the present disclosure is an exercise support system including: the exercise support device according to the first aspect; and a terminal including a transmission unit that transmits information indicating the physical quantity to the acquisition unit of the exercise support device.

A third aspect of the present disclosure is an exercise support method including: acquiring a physical quantity indicating an execution degree of an exercise according to an exercise program indicating a predetermined self-weight exercise; deriving a subsequent intensity of the exercise by using the acquired physical quantity; and presenting the derived intensity.

A fourth aspect of the present disclosure is a program for causing a computer to execute a process of: acquiring a physical quantity indicating an execution degree of an exercise according to an exercise program indicating a predetermined self-weight exercise; deriving a subsequent intensity of the exercise using the acquired physical quantity; and presenting the derived intensity.

Advantageous Effects of Invention

As described above, according to the technique of the present disclosure, it is possible to appropriately set the intensity of a self-weight exercise in the resistance exercise.

BRIEF DESCRIPTION OF DRAWINGS

Fig. 1 is a block diagram illustrating an example of a hardware configuration of an exercise support system according to an embodiment.
Fig. 2 is a block diagram illustrating an example of a functional configuration of the exercise support system according to the embodiment.
Fig. 3 is a diagram illustrating an example of a worn state of a wearable terminal according to the embodiment.
Fig. 4 is a diagram illustrating another example of a worn state of the wearable terminal according to the embodiment.
Fig. 5 is a diagram for explaining intensity of a self-weight exercise (push-up) according to the embodiment.
Fig. 6 is a diagram for explaining intensity of a self-weight exercise (leg-raise) according to the embodiment.
Fig. 7 is a schematic diagram illustrating an example of a configuration of an exercise program database according to the embodiment.
Fig. 8 is a schematic diagram illustrating an example of a configuration of an exercise history information database according to the embodiment.
Fig. 9 is a schematic diagram illustrating an example of a configuration of an intensity information database according to the embodiment.
Fig. 10 is a flowchart illustrating an example of a registration request process according to the embodiment.
Fig. 11 is a front view illustrating an example of a configuration of a registration screen according to the embodiment.
Fig. 12 is a flowchart illustrating an example of a registration execution process according to the embodiment.
Fig. 13 is a flowchart illustrating an example of an exercise execution process according to the embodiment.
Fig. 14 is a front view illustrating an example of a configuration of an exercise program display screen according to the embodiment.
Fig. 15 is a flowchart illustrating an example of an exercise status registration process according to the embodiment.
Fig. 16 is a flowchart illustrating an example of an intensity setting process according to the embodiment.
Fig. 17 is a schematic diagram illustrating an example of a configuration of an exercise program creation model according to the embodiment.

DESCRIPTION OF EMBODIMENTS

Hereinafter, exemplary embodiments for carrying out the technique of the present disclosure will be described in detail with reference to the drawings. Note that, in the present embodiment, a case will be described where the technique of the present disclosure is applied to an exercise support system including an exercise support device configured by a server computer and the like, a plurality of mobile terminals that are terminals individually used by users, and a plurality of wearable terminals that are worn by the users when each user perform exercise according to the exercise program. Furthermore, in the present embodiment, an exemplary embodiment in a case where the technique of the present disclosure is applied for a case where the user is a patient with type 2 diabetes will be described.

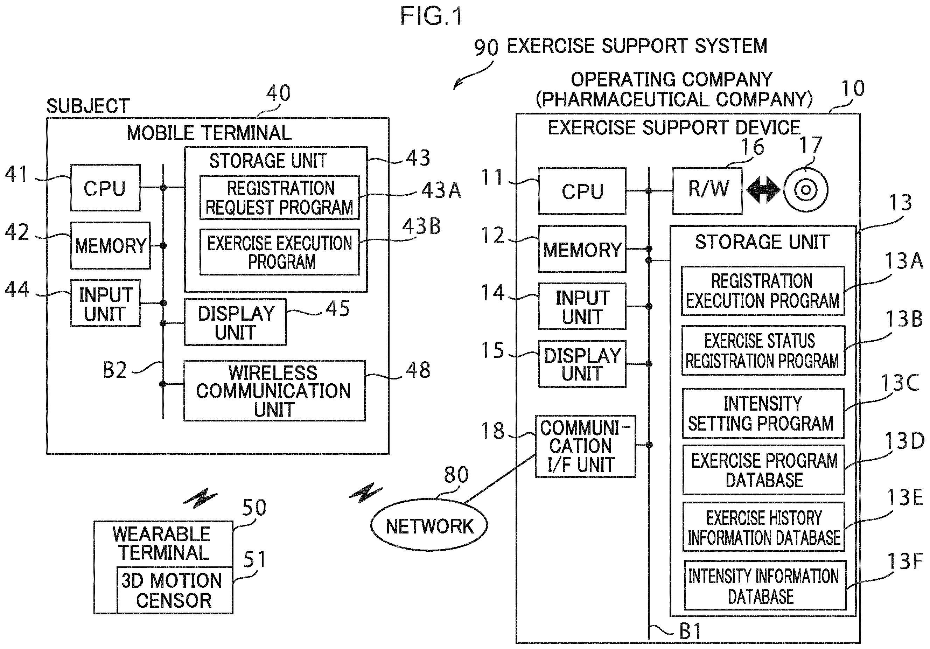

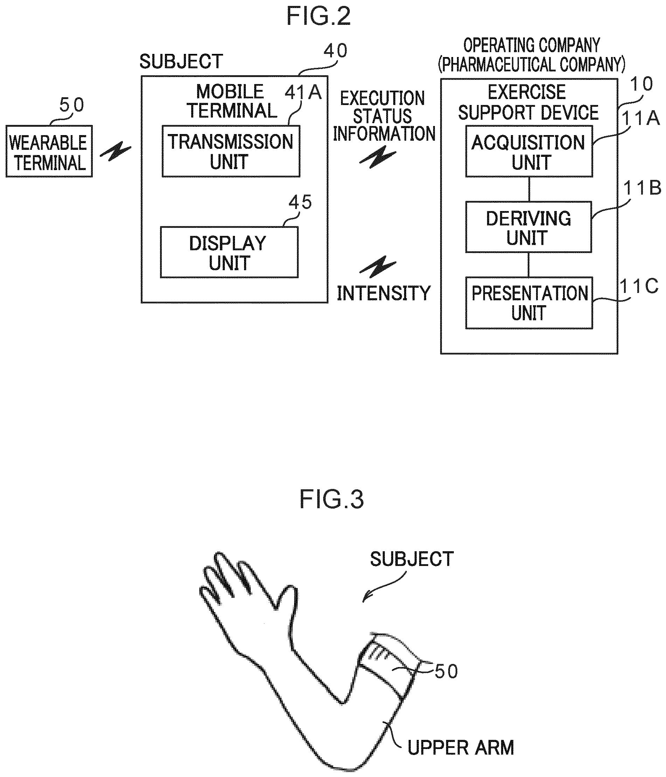

First, a configuration of an exercise support system 90 according to the present embodiment will be described with reference to Figs. 1 and 2. As illustrated in Fig. 1, an exercise support system 90 according to the present embodiment includes an exercise support device 10 and a plurality of mobile terminals 40, each of which can access a network 80. Note that examples of the exercise support device 10 include general-purpose information processing devices such as a personal computer and a server computer. In addition, examples of the mobile terminals 40 include portable and wirelessly communicable terminals such as a smartphone, a tablet terminal, a personal digital assistant (PDA), and a notebook-type personal computer.

In addition, the exercise support system 90 according to the present embodiment includes a plurality of wearable terminals 50. Note that examples of the wearable terminals 50 include various terminals having a three-dimensional (3-Dimensional) motion sensor including an acceleration sensor and a gyro sensor, such as a wristband-type terminal, a watch-type terminal, a clip-type terminal, and a glasses-type terminal. Hereinafter, a case where a wristband-type terminal is applied as the wearable terminals 50 will be described.

The exercise support device 10 according to the present embodiment is a device managed by an operating company (in the present embodiment, a pharmaceutical company; hereinafter, also simply referred to as an "operating company") of the exercise support system 90. The exercise support device 10 includes a central processing unit (CPU) 11, a memory 12 as a temporary storage area, a nonvolatile storage unit 13, an input unit 14 such as a keyboard and a mouse, a display unit 15 such as a liquid crystal display, a medium read/write device (R/W) 16, and a communication interface (I/F) unit 18. The CPU 11, the memory 12, the storage unit 13, the input unit 14, the display unit 15, the medium read/write device 16, and the communication I/F unit 18 are connected to each other via a bus B1. The medium read/write device 16 reads information written in a recording medium 17 and writes information in the recording medium 17.

The storage unit 13 is realized by a hard disk drive (HDD), a solid state drive (SSD), a flash memory, or the like. The storage unit 13 as a storage medium stores a registration execution program 13A, an exercise status registration program 13B, and an intensity setting program 13C. The recording medium 17 in which the registration execution program 13A, the exercise status registration program 13B, and the intensity setting program 13C are written is set in the medium read/write device 16, and the medium read/write device 16 reads the registration execution program 13A, the exercise status registration program 13B, and the intensity setting program 13C from the recording medium 17, whereby the registration execution program 13A, the exercise status registration program 13B, and the intensity setting program 13C are stored in the storage unit 13. The CPU 11 reads the registration execution program 13A, the exercise status registration program 13B, and the intensity setting program 13C from the storage unit 13, develops them in the memory 12, and sequentially executes the processes of the registration execution program 13A, the exercise status registration program 13B, and the intensity setting program 13C.

In addition, the storage unit 13 stores an exercise program database 13D, an exercise history information database 13E, and an intensity information database 13F. Details of the exercise program database 13D, the exercise history information database 13E, and the intensity information database 13F will be described later.

On the other hand, the mobile terminals 40 according to the present embodiment are terminals possessed by each of a plurality of users (in the present embodiment, patients with type 2 diabetes; hereinafter, also simply referred to as "subjects") who are expected to use services (a service that supports exercise according to an exercise program, and hereinafter referred to as a "provided service") provided by the exercise support system 90. Each of the mobile terminals 40 includes a CPU 41, a memory 42 as a temporary storage area, a nonvolatile storage unit 43, an input unit 44 such as a touch panel, a display unit 45 such as a liquid crystal display, and a wireless communication unit 48 that performs wireless communication according to a predetermined communication standard (In the present embodiment, two types of communication standards, Wi-Fi (registered trademark) and Bluetooth (registered trademark), are used.). The CPU 41, the memory 42, the storage unit 43, the input unit 44, the display unit 45, and the wireless communication unit 48 are connected to each other via a bus B2.

The storage unit 43 is realized by an HDD, an SSD, a flash memory, or the like. The storage unit 43 as a storage medium stores a registration request program 43A and an exercise execution program 43B. The registration request program 43A and the exercise execution program 43B are installed in the storage unit 43 in advance. The CPU 41 reads the registration request program 43A and the exercise execution program 43B from the storage unit 43, develops the programs in the memory 42, and sequentially executes the processes of the registration request program 43A and the exercise execution program 43B.

Incidentally, the exercise program according to the present embodiment is a program with a self-weight exercise in resistance exercise. Note that examples of the self-weight exercise according to the present embodiment include push-up, leg-raise, lunge, and self-weight squat.

In the present embodiment, frequency, intensity, duration, and type of a self-weight exercise included in the exercise program are defined, but the present invention is not limited thereto. For example, the self-weight exercise may include intensity among frequency, intensity, duration, and type, and two to three types of combinations may be defined. Alternatively, at least one of the frequency, the intensity, the duration, and the type of the self-weight exercise may be defined.

The wearable terminals 50 according to the present embodiment are worn by each subject when the subject performs exercise according to an exercise program. Note that the wearable terminals 50 according to the present embodiment have a function of transmitting a physical quantity (in the present embodiment, a physical quantity (In the present embodiment, both the amount and the speed of an exercise with respect to the three-dimensional direction are included. Hereinafter, they are also simply referred to as "execution status information".) indicating an execution degree of the exercise performed by each wearer (subject)) detected by a built-in 3D motion sensor 51 to each mobile terminal 40 possessed by the subject by wireless communication according to a predetermined communication standard (in the present embodiment, Bluetooth (registered trademark)).

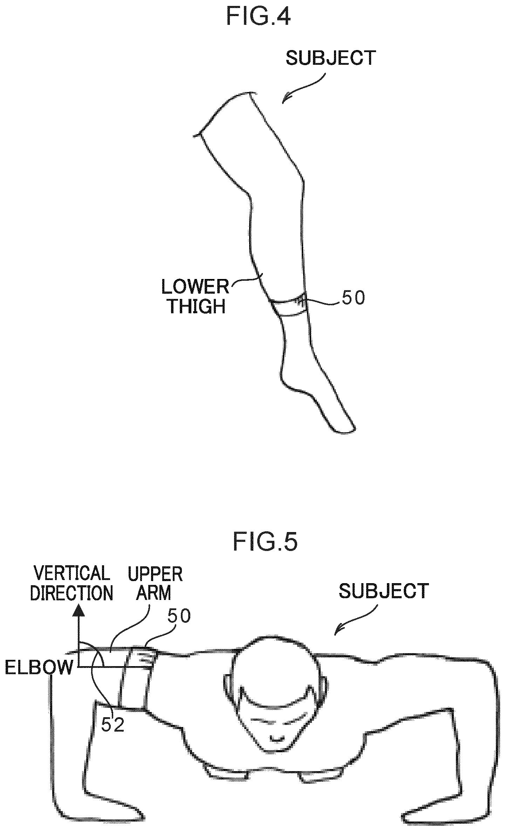

Figs. 3 and 4 illustrate an example of a worn state of the wearable terminal 50 for the subject in a case where the wristband-type terminal is applied as the wearable terminal 50. Note that Fig. 3 illustrates an example of a case where an exercise to be performed is push-up, and the example illustrated in Fig. 3 illustrates an example of a case where the wearable terminal 50 is worn on the upper arm of an arm of the subject. Furthermore, Fig. 4 illustrates an example of a case where an exercise to be performed is leg-raise, and the example illustrated in Fig. 4 illustrates an example of a case where the wearable terminal 50 is worn on the lower thigh of a leg of the subject. However, the wearing position of the wearable terminal 50 is not limited to these examples, and for example, in a case where an exercise to be performed is leg-raise, the wearable terminal 50 may be worn on the thigh of a leg of the subject in addition to the lower thigh.

Next, functional configurations of the exercise support device 10 and the mobile terminal 40 according to the present embodiment will be described with reference to Fig. 2. As illustrated in Fig. 2, the exercise support device 10 according to the present embodiment includes an acquisition unit 11A, a deriving unit 11B, and a presentation unit 11C. The CPU 11 of the exercise support device 10 executes the exercise status registration program 13B and the intensity setting program 13C to function as the acquisition unit 11A, the deriving unit 11B, and the presentation unit 11C.

The acquisition unit 11A according to the present embodiment acquires a physical quantity indicating an execution degree of an exercise according to the exercise program indicating a predetermined self-weight exercise. In the present embodiment, the execution status information detected by the wearable terminal 50 when the subject performs a self-weight exercise included in the exercise program is applied as a physical quantity indicating the execution degree of the exercise.

On the other hand, the deriving unit 11B derives intensity of the subsequent self-weight exercise using the physical quantity (execution status information) acquired by the acquisition unit 11A. The deriving unit 11B according to the present embodiment derives intensity of a next self-weight exercise using the physical quantity acquired immediately before by the acquisition unit 11A, but the present invention is not limited thereto. For example, the deriving unit 11B may derive intensity of the subsequent self-weight exercise by using a physical quantity or the like acquired a predetermined number of times (as an example, one time) before the physical quantity acquired immediately before. Further, for example, the deriving unit 11B may derive intensity of the self-weight exercise after a predetermined number of times (as an example, one time) from the next time. Hereinafter, the intensity according to the present embodiment will be specifically described.

For example, in a case where the push-up is performed as the self-weight exercise, as illustrated in Fig. 5 as an example, an angle 52 of the upper arm of the subject changes with an elbow as a rotation center, along a vertical direction as a reference direction. Here, as the angle 52 in the middle of performing the push-up is closer to 180 degrees, the load on muscles of the upper arm and greater pectoralis becomes larger. In the exercise support system 90 according to the present embodiment, the angle 52 itself at the time of performing the push-up obtained using the execution status information obtained from the wearable terminal 50 is applied as the intensity against the push-up.

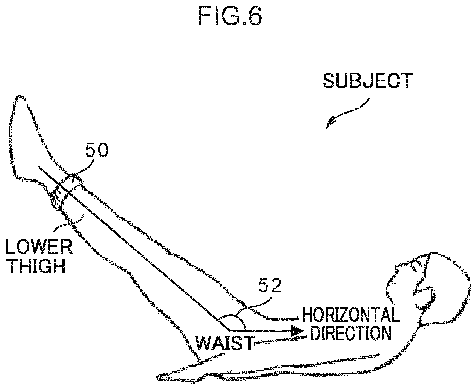

Similarly, in a case where the leg-raise is performed as the self-weight exercise, as illustrated in Fig. 6 as an example, an angle 52 of a lower thigh of the subject changes with a waist as the rotation center and a horizontal direction as the reference direction. Here, a load is applied to abdominal muscles depending on the angle 52 during the execution of the leg-raise. In the exercise support system 90 according to the present embodiment, the angle 52 itself obtained by using the execution status information obtained from the wearable terminal 50 at the time of performing the leg-raise is applied as the intensity against the leg-raise. Note that the intensity of other self-weight exercises is defined by the same concept, but the description thereof is omitted here. In deriving the intensity of each self-weight exercise, for example, a physical quantity serving as a reference of the corresponding self-weight exercise such as 90 degrees as an angle may be used as a "reference physical quantity".

In addition, in a case where the self-weight exercise to be performed is a set exercise by a plurality of repetitive executions, the deriving unit 11B according to the present embodiment derives intensity using the physical quantity acquired by the acquisition unit 11A for at least one latest set of exercises as a physical quantity acquired immediately before. In addition, in a case where the self-weight exercise to be executed is a set exercise by a plurality of repetitive executions, the deriving unit 11B according to the present embodiment derives intensity of a next set exercise as intensity of the next self-weight exercise.

For example, if the self-weight exercise to be performed is an exercise in which one set is an exercise in which 10 push-ups are performed, and is performed every day with 3 sets per day, the intensity of at least the first push-up of a next day is derived using a physical quantity (execution status information) obtained at the time of performing a third push-up of a previous day.

Note that the deriving unit 11B according to the present embodiment derives intensity as any one of a value increased from a previous value (hereinafter, referred to as an "increase value"), a value decreased from the previous value (hereinafter, referred to as a "decrease value"), and a same value as the previous value (hereinafter, referred to as "the same value"). However, the present invention is not limited to this mode, and the deriving unit 11B may apply only a combination of the increase value and the same value among the increase value, the decrease value, and the same value, may apply only a combination of the increase value and the decrease value, or may apply only the increase value.

In addition, the deriving unit 11B according to the present embodiment derives the intensity of a subsequent self-weight exercise according to whether or not the intensity indicated by the physical quantity acquired by the acquisition unit 11A is equal to or greater than a success threshold set in advance as a lower limit value of a range considered to be successful.

Then, the presentation unit 11C presents intensity derived by the deriving unit 11B. Note that, in the present embodiment, presentation using the display unit 45 of the mobile terminal 40 is applied as a presentation of the intensity by the presentation unit 11C, but the present invention is not limited thereto. For example, other presentation such as presentation by voice using a speaker or presentation by printing using a printing apparatus may be applied as presentation by the presentation unit 11C.

On the other hand, the mobile terminal 40 according to the present embodiment includes a transmission unit 41A. The CPU 41 of the mobile terminal 40 executes the exercise execution program 43B to function as the transmission unit 41A.

The transmission unit 41A according to the present embodiment transmits information (execution status information) indicating the physical quantity to the acquisition unit 11A of the exercise support device 10.

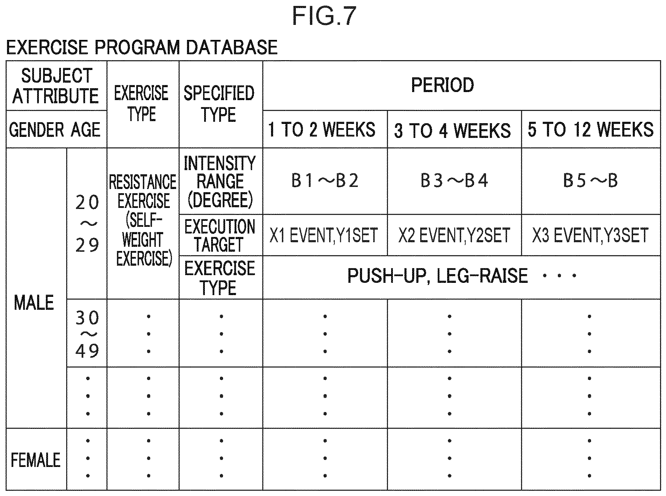

Next, the exercise program database 13D according to the present embodiment will be described with reference to Fig. 7.

As illustrated in Fig. 7, the exercise program database 13D according to the present embodiment stores exercise program information including a combination of a predetermined type of self-weight exercise in resistance exercise for each subject attribute indicating the attribute of the subject (in the present embodiment, gender and age). In the example illustrated in Fig. 7, a specified type (in the present embodiment, the intensity range which is a success range of the intensity, the execution target indicating the execution frequency and the like, and the exercise type indicating the type of the exercise) for each predetermined period (in the example illustrated in Fig. 7, a period of three stages of 1 to 2 weeks, 3 to 4 weeks, and 5 to 12 weeks) is stored as the exercise program information. Hereinafter, with respect to the predetermined period, 1 to 2 weeks are referred to as a "first period", 3 to 4 weeks are referred to as a "second period", and 5 to 12 weeks are referred to as a "third period".

In the example illustrated in Fig. 7, for example, as the exercise program information corresponding to men from 20 to 29 years of age, B1 to B2 are applied as the intensity range in the first period, X1 event and Y1 set are applied as the execution target, and push-up, legraise, and the like are applied as the exercise type. Note that, here, B1, B2, X1, Y1, and the like are described as each information of the intensity range and the execution target in the specified type for convenience, but it goes without saying that specific numerical values are actually stored. As described above, a lower limit value and an upper limit value in the intensity range are the lower limit value and the upper limit value of a measurement value (in the present embodiment, the angle) actually obtained when the self-weight exercise is performed.

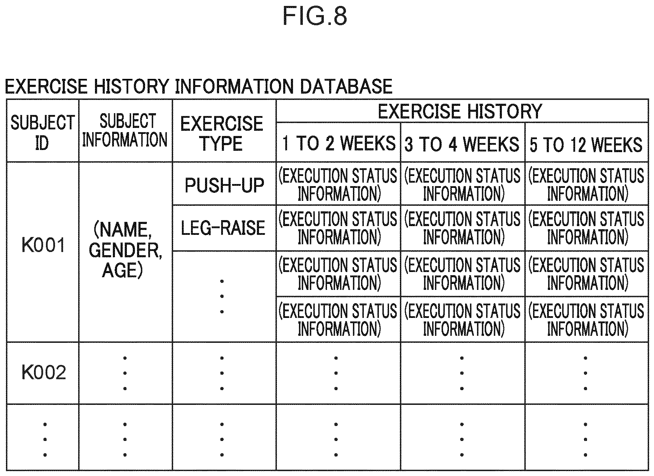

Next, the exercise history information database 13E according to the present embodiment will be described with reference to Fig. 8.

As illustrated in Fig. 8, the exercise history information database 13E according to the present embodiment stores each piece of information of a subject identification (ID), subject information, an exercise type, and an exercise history for each predetermined period (in the present embodiment, three stages of a first period, a second period, and a third period are described above.). In addition, the subject information includes each piece of information of name, gender, and age.

The subject ID is information assigned in advance for each subject to identify each subject, the name is information indicating a name of a corresponding subject, the gender is information indicating a gender of the corresponding subject, and the age is information indicating an age of the corresponding subject. In addition, the exercise type is information indicating the type of a self-weight exercise performed by the corresponding subject according to the presented exercise program. In addition, the exercise history is information indicating a situation in which the corresponding subject performs the self-weight exercise of the corresponding exercise type in the corresponding period, and in the present embodiment, the above-described execution status information is applied. However, the present invention is not limited to this embodiment, and instead of the execution status information, an embodiment may be employed in which the intensity when the corresponding self-weight exercise is executed is applied as the exercise history.

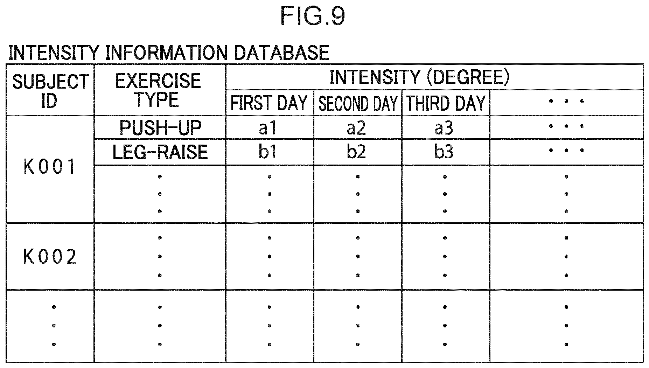

Next, the intensity information database 13F according to the present embodiment will be described with reference to Fig. 9.

As illustrated in Fig. 9, the intensity information database 13F according to the present embodiment stores each information of the subject ID, the exercise type, and the intensity for each day in the predetermined period (in the present embodiment, three stages of the first period, the second period, and the third period described above.).

The subject ID is the same information as the subject ID in the exercise history information database 13E, and the exercise type is also the same information as the exercise type in the exercise history information database 13E. In addition, the intensity is information indicating a lower limit value (hereinafter, referred to as a "success threshold") of a success range of the intensity when the corresponding subject performs the corresponding self-weight exercise in the corresponding exercise schedule.

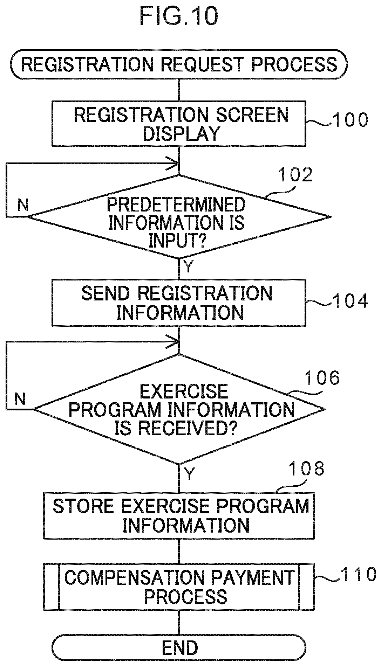

Next, the operation of the exercise support system 90 according to the present embodiment will be described with reference to Figs. 10 to 16. Note that, in the following, a case where the exercise program database 13D is constructed in advance in order to avoid complication will be described.

First, the operation of the mobile terminal 40 according to the present embodiment when the subject requests the operating company to register the subject himself/herself in the provided service will be described with reference to Figs. 10 to 11. When the CPU 41 of any one of the mobile terminals 40 executes the registration request program 43A, the registration request process illustrated in Fig. 10 is executed. The registration request processing illustrated in Fig. 10 is executed, for example, in a case where an execution instruction of the registration request process is input from a patient with type 2 diabetes who desires to use the provided service via the input unit 44 of his/her mobile terminal 40.

In Step 100 of Fig. 10, the CPU 41 performs control to display a registration screen having a predetermined configuration on the display unit 45, and then in Step 102, the CPU 41 waits until predetermined information is input.

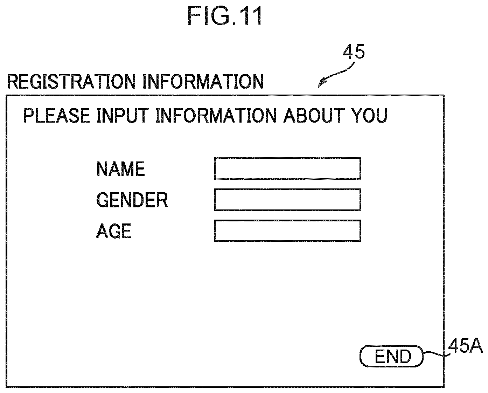

Fig. 11 illustrates an example of a registration screen according to the present embodiment. As illustrated in Fig. 11, on the registration screen according to the present embodiment, a message prompting input of information regarding the subject himself/herself is displayed, and an input area for inputting the name, gender, and age of the subject himself/herself is displayed.

When the registration screen illustrated in Fig. 11 is displayed on the display unit 45, the subject inputs each piece of information regarding the subject to the corresponding input area via the input unit 44, and then designates an end button 45A via the input unit 44. If the end button 45A is designated by the subject, an affirmative determination is made in Step 102, the processing proceeds to Step 104.

In Step 104, the CPU 41 transmits each piece of information (hereinafter, referred to as "registration information") input by the subject on the registration screen to the exercise support device 10 via the wireless communication unit 48 and the network 80. When the registration information is received, the exercise support device 10 transmits the exercise program information corresponding to the received registration information to the mobile terminal 40 which is a transmission source of the registration information as to be described later.

Therefore, in Step 106, the CPU 41 waits until the exercise program information is received from the exercise support device 10, and in Step 108, the CPU 41 stores the exercise program information received from the exercise support device 10 in the storage unit 43. By storing the exercise program information, the subject can refer to the exercise program indicated by the exercise program information at any time.

Meanwhile, after transmitting the exercise program information to the mobile terminal 40, the exercise support device 10 according to the present embodiment executes processing (hereinafter, referred to as "compensation payment instruction process") of instructing the subject to pay compensation for the provision of the exercise program information. Therefore, in Step 110, the CPU 41 executes a process for paying the compensation to the operating company (hereinafter, referred to as "compensation payment processing") according to the compensation payment instruction process, and thereafter, ends the present registration request process. Note that, as will be described later, the exercise support device 10 according to the present embodiment transmits the subject ID newly given to the subject to the mobile terminal 40 of the subject when executing the compensation payment instruction process. Therefore, in Step 110, the CPU 41 executes compensation payment processing and also executes a process of storing the received subject ID in the storage unit 43.

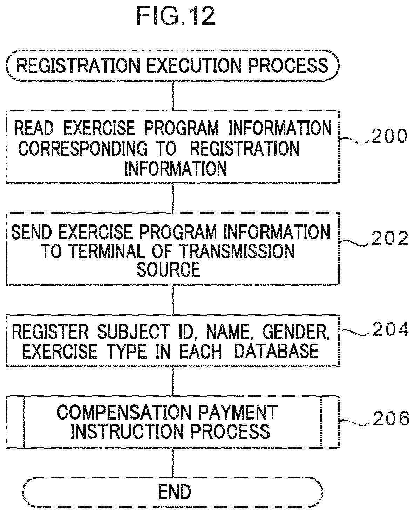

Next, with reference to Fig. 12, an operation of the exercise support device 10 according to the present embodiment when a request for own registration for the provided service is received from any subject will be described. When the CPU 11 of the exercise support device 10 executes the registration execution program 13A, the registration execution process illustrated in Fig. 12 is executed. The registration execution process illustrated in Fig. 12 is executed when the above-described registration information is received from the mobile terminal 40 possessed by any of the subjects.

In Step 200 of Fig. 12, the CPU 11 reads the exercise program information corresponding to the gender and the age of the subject in the received registration information from the exercise program database 13D. In Step 202, the CPU 11 transmits the read exercise program information to the mobile terminal 40 that is the transmission source of the received registration information via the communication I/F unit 18 and the network 80.

In Step 204, the CPU 11 newly generates a subject ID that is not registered in the exercise history information database 13E at that time. The CPU 11 stores (registers) the subj ect ID, the respective pieces of information of name, gender, and age in the received registration information, and the exercise type in the transmitted exercise program information in the exercise history information database 13E in association with each other. In Step 204, the CPU 11 stores (registers) the generated subject ID and the exercise type in the transmitted exercise program information in the intensity information database 13F in association with each other.

In Step 206, the CPU 11 executes the compensation payment instruction process described above, and then ends the present registration execution process. When executing the compensation payment instruction process, the CPU 11 transmits the subject ID generated in the process of Step 204 to the mobile terminal 40 that is the transmission source of the received registration information via the communication I/F unit 18 and the network 80.

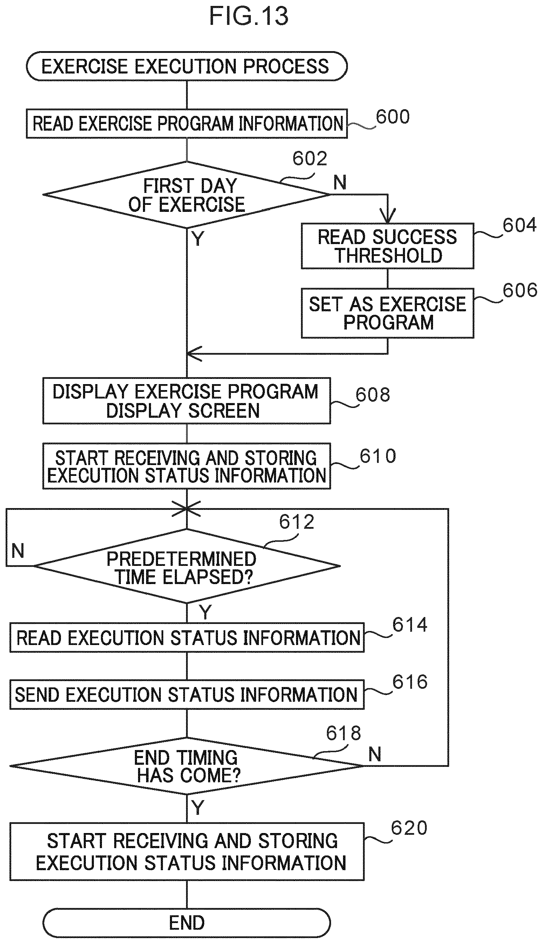

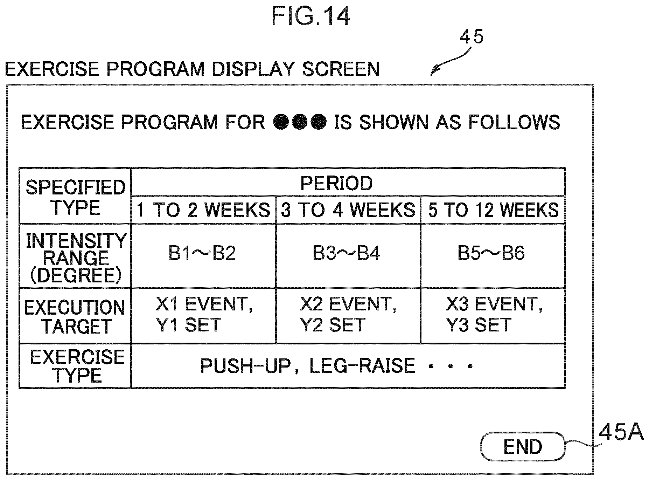

Next, the operation of the mobile terminal 40 when the subject performs the self-weight exercise indicated by the exercise program information received from the exercise support device 10 will be described with reference to Figs. 13 and 14. When the CPU 41 of the mobile terminal 40 executes the exercise execution program 43B, the exercise execution processing illustrated in Fig. 13 is executed. The exercise execution process illustrated in Fig. 13 is executed when an instruction to execute the exercise execution process is input from the subject via the input unit 44. Note that, at this time, the subject wears the wearable terminal 50 possessed by the subject and enters an operating state. As a result, the mobile terminal 40 can continuously receive the execution status information described above from the wearable terminal 50.

In Step 600 of Fig. 13, the CPU 41 reads the exercise program information stored in the registration request process from the storage unit 43. In Step 602, the CPU 41 determines whether or not it is a first day to perform the self-weight exercise indicated by the exercise program information read out on that day, and if an affirmative determination is made, the process proceeds to Step 608 as to be described later. On the other hand, if a negative determination is made, the CPU 41 proceeds to Step 604. Note that, in the present embodiment, the determination as to whether or not it is the first day to perform the self-weight exercise is made by causing the subject to input, via the input unit 44, whether today is how many days to perform the self-weight exercise since a first day on which the self-weight exercise was performed, but the present invention is not limited thereto. For example, it may be determined whether or not it is the first day from the registration status of the execution status information with reference to the exercise history information database 13E.

By the way, as will be described later, when the subject performs the self-weight exercise indicated by the exercise program information over the predetermined period, the exercise support device 10 according to the present embodiment derives a success threshold which is a lower limit value of a success range of the intensity of the self-weight exercise on and after the second day, and registers the success threshold in the intensity information database 13F. The success threshold is derived using the execution status information of the same type of self-weight exercise of the previous day.

Therefore, in Step 604, the CPU 41 reads the current success threshold registered by the exercise support device 10 from the intensity information database 13F via the wireless communication unit 48 and the network 80. In Step 606, the CPU 41 replaces the lower limit value of the intensity range of the corresponding period in the read exercise program information with the read success threshold, and then proceeds to Step 608.

In Step 608, the CPU 41 performs control to display an exercise program display screen having a predetermined configuration on the display unit 45 using the exercise program information subjected to the above processing.

Fig. 14 illustrates an example of an exercise program display screen according to the present embodiment. As illustrated in Fig. 14, the exercise program indicated by the exercise program information suitable for the subject is displayed on the exercise program display screen according to the present embodiment. Note that the example illustrated in Fig. 14 illustrates a case where the lower limit value of the intensity range is not replaced by the processing of Steps 604 to 606. However, when the replacement is performed, the exercise program in which the lower limit value of the corresponding intensity range is changed is displayed. By referring to the displayed exercise program, the subject can grasp the type, the number of times, and the like of the self-weight exercise that the subject performs, and can grasp the lower limit value of an allowable range of the intensity that can be regarded as success in performing the self-weight exercise. Therefore, the subject can perform an effective self-weight exercise by setting the grasped lower limit value as a target of the intensity when performing the corresponding self-weight exercise.

In Step 610, the CPU 41 starts receiving the execution status information from the wearable terminal 50 and storing the received execution status information in the storage unit 43. In Step 612, the CPU 41 waits until a predetermined time (in the present embodiment, 10 seconds) elapses, and in Step 614, the transmission unit 41A reads, from the storage unit 43, the execution status information stored from the predetermined time point going back from this time point to this time point.

In Step 616, the transmission unit 41A reads the subject ID of the subject from the storage unit 43, and transmits the read execution status information together with the read subject ID to the exercise support device 10 via the wireless communication unit 48 and the network 80. In Step 618, the CPU 41 determines whether or not a predetermined end timing has come, and if a negative determination is made, the process returns to Step 612. On the other hand, if an affirmative determination is made, the CPU 41 proceeds to Step 620. In the present embodiment, a timing at which the operating state of the wearable terminal 50 worn by the subject is stopped is applied as the end timing, but the present invention is not limited thereto. For example, a timing at which the subject inputs an instruction to end the exercise execution process via the input unit 44 may be set as the end timing.

In Step 620, the CPU 41 stops receiving the execution status information started by the processing in Step 610 and storing it in the storage unit 43, and then the present exercise execution process ends.

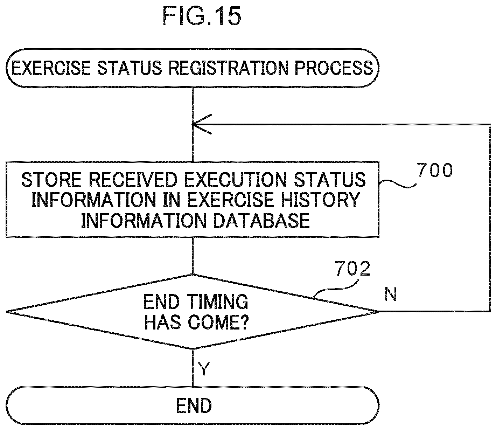

Next, an operation of the exercise support device 10 in a case where the exercise status registration process according to the present embodiment is executed will be described with reference to Fig. 15. When the CPU 11 of the exercise support device 10 executes the exercise status registration program 13B, the exercise status registration process illustrated in Fig. 15 is executed. The exercise status registration processing illustrated in Fig. 15 is executed when the reception of the execution status information is started from the mobile terminal 40 of any registered subject.

In Step 700 of Fig. 15, the CPU 11 stores the received execution status information in the exercise history information database 13E as the exercise history corresponding to the subject ID received together with the execution status information. In Step 702, the CPU 11 determines whether or not a predetermined end timing has come, and if a negative determination is made, the process returns to Step 700. On the other hand, in a case where an affirmative determination is made, the CPU 41 ends the present exercise state registration process. In the present embodiment, as the end timing, a timing at which the execution status information from the corresponding mobile terminal 40 has not been received over a predetermined time period is applied, but the present invention is not limited thereto. For example, the exercise program information provided to the subject corresponding to the received subject ID is referred. A timing or the like at which storing the execution status information in the exercise history information database 13E is completed by an amount corresponding to the amount of the corresponding self-weight exercise for one day indicated by the exercise program information may be set as the end timing.

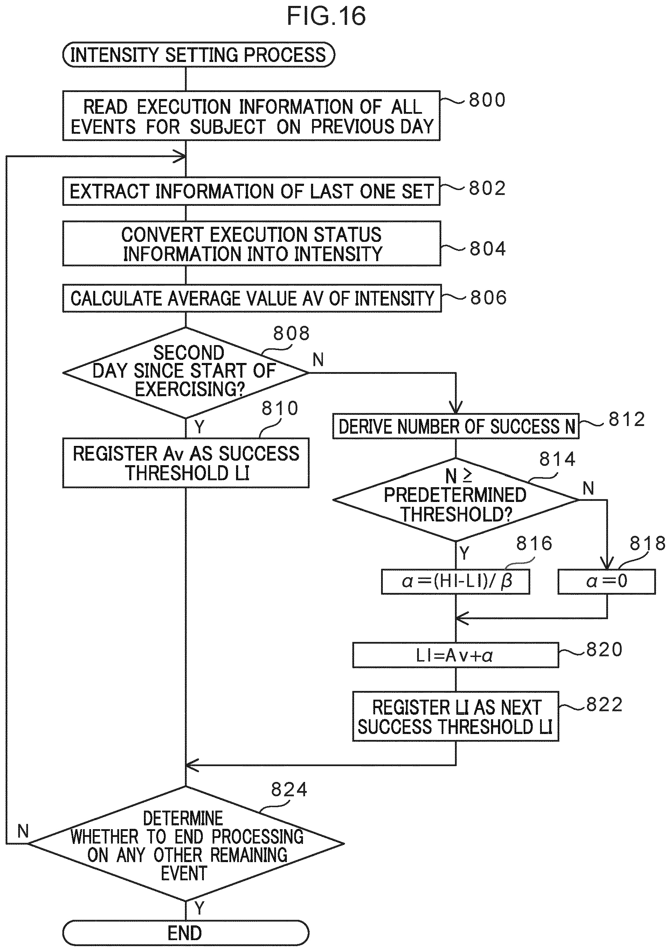

Next, the action of the exercise support device 10 in a case of executing the intensity setting process according to the present embodiment will be described with reference to Fig. 16. The CPU 11 of the exercise support device 10 executes the intensity setting program 13C to perform the intensity setting process illustrated in Fig. 16. The intensity setting process illustrated in Fig. 16 is executed for each registered subject at a predetermined time (In the present embodiment, the time is midnight.) for each day from the second day since the start of the self-weight exercise according to the exercise program information transmitted to the subject and until the end of the execution period of the self-weight exercise for each registered subject.

In Step 800 of Fig. 16, the acquisition unit 11A reads the execution status information of a previous day corresponding to the processing target person (hereinafter, referred to as a "processing subject") and corresponding to all the exercise types performed by the processing subject from the exercise history information database 13E. In Step 802, the deriving unit 11B extracts, from the read execution status information, execution status information for one event of the self-weight exercise to be executed and for the last one set of the previous day. Note that the self-weight exercise from which the execution status information is extracted is hereinafter referred to as a "processing target exercise".

In Step 804, the deriving unit 11B derives the intensity of the processing target exercise for each time of the self-weight exercise as described above using the execution status information of one set extracted with respect to the processing target exercise. In Step 806, the deriving unit 11B calculates an average value Av of the derived intensities for one set. In Step 808, the deriving unit 11B determines whether or not this time point is the second day since the exercise start date by the processing subject, and proceeds to Step 810 if the determination is affirmative.

In Step 810, the presentation unit 11C stores the average value Av as the success threshold LI, which is the lower limit value of the success range of the intensity of the subsequent self-weight exercise, in the corresponding area of the intensity information database 13F, and then proceeds to Step 824.

On the other hand, if a negative determination is made in Step 808, that is, if this time point is on or after the third day since the exercise start date by the processing subject, the process proceeds to Step 812. In Step 812, the deriving unit 11B derives the number of times of the intensity equal to or higher than the success threshold LI registered in the intensity information database 13F as the previous day among the intensities for one set derived by the process in Step 804, that is, the number of times (Hereinafter, the number of times of success is referred to as "number of times of success".) N that the processing target exercise is successful.

In Step 814, the deriving unit 11B determines whether or not the derived number of successes N is greater than or equal to a predetermined threshold value, and proceeds to Step 816 if an affirmative determination is made. The deriving unit 11B sets an upper limit threshold of the corresponding intensity to HI, applies the success threshold LI for a previous day as the success threshold LI, and calculates an adjustment value α by the following Formula (1) with β as a predetermined coefficient (as an example, 20). Thereafter, the deriving unit 11B proceeds to Step 820 as to be described later. Here, as an upper limit threshold HI, the upper limit value of the intensity range in the corresponding period of the corresponding exercise program information may be applied. In addition, the coefficient β greatly affects the adjustment value α as is clear from Formula (1). Therefore, it is preferable to set in advance according to the execution period of the corresponding self-weight exercise by the applied exercise program, the level of the effect required for the self-weight exercise to be executed, and the like.

$$\alpha = (HI - LI)/\beta \quad (1)$$

On the other hand, if a negative determination is made in Step 814, that is, if the number of successes N is less than the predetermined threshold, the process proceeds to Step 818. After setting the adjustment value α to 0 (zero), the deriving unit 11B proceeds to Step 820.

In Step 820, the deriving unit 11B calculates a success threshold LI to be applied to a next exercise to be processed by the following Formula (2). In Step 822, the presentation unit 11C stores (registers) the calculated success threshold LI in the corresponding area of the intensity information database 13F, and then proceeds to Step 824.

$$LI = Av + \alpha \quad (2)$$

In Step 824, the deriving unit 11B determines whether or not there remains an exercise type for which the above processing has not been executed among the respective exercise types of the self-weight exercise from which the execution status information has been read in Step 800. If a negative determination is made, the process returns to Step 802, and on the other hand, when an affirmative determination is made, this intensity setting process ends. Note that, when the processing of Steps 802 to 824 is repeatedly executed, the self-weight exercise of the exercise type that has not been set as the processing target exercise is set as a processing target exercise.

As described above, according to the present embodiment, the exercise support device 10 includes the acquisition unit 11A that acquires a physical quantity indicating the execution degree of an exercise according to the exercise program indicating the predetermined self-weight exercise, the deriving unit 11B that derives intensity of a subsequent exercise using the physical quantity acquired by the acquisition unit 11A, and the presentation unit 11C that presents the intensity derived by the deriving unit 11B. Therefore, it is possible to appropriately set the intensity against the self-weight exercise in the resistance exercise.

In addition, according to the present embodiment, the frequency, intensity, duration, and type of the self-weight exercise are defined. Therefore, the self-weight exercise can be defined more simply and quantitatively. Note that, in the present embodiment, each piece of information of the execution target in the exercise program database 13D is applied as an example of the information regarding the frequency and the duration.

In addition, according to the present embodiment, the intensity is derived using the physical quantity acquired immediately before by the acquisition unit 11A. Therefore, it is possible to set the intensity more suitable for the actual situation.

Furthermore, according to the present embodiment, in a case where the self-weight exercise is a set exercise by a plurality of repetitive executions, the intensity is derived using the physical quantity acquired for at least one latest set of movements as the physical quantity acquired immediately before. Therefore, even in a case where the exercise is a set exercise, it is possible to set the intensity more appropriate to an actual situation.

Furthermore, according to the present embodiment, intensity of a next exercise is derived by the deriving unit 11B. Therefore, the intensity can be appropriately set for the next self-weight exercise.

In addition, in a case where the self-weight exercise to be executed is a set exercise by a plurality of repetitive executions, the deriving unit 11B according to the present embodiment derives intensity of a next set exercise as intensity of the next self-weight exercise. Therefore, even when an exercise is the set exercise, the intensity of the next self-weight exercise can be appropriately set.

Furthermore, according to the present embodiment, the deriving unit 11B derives the intensity as any one of a value increased from the previous value, a value decreased from the previous value, and the same value as the previous value. Therefore, the intensity can be appropriately set without any limitation according to the situation of the exercise, the physical strength, and the like of an individual.

Further, according to the present embodiment, in the deriving unit 11B, the self-weight exercise is a set exercise by a plurality of repetitive executions. Therefore, it is possible to appropriately set the intensity with respect to the self-weight exercise set as the set exercise.

According to the present embodiment, the acquisition unit 11A acquires the physical quantity as a value indicating both the amount and the speed of the exercise in the three-dimensional direction when the exercise is performed. Therefore, as a result of being able to derive the intensity using elements (the amount and speed of exercise) important for defining the intensity of the exercise, the intensity can be set more accurately.

Furthermore, according to the present embodiment, the acquisition unit 11A acquires the physical quantity using the wearable terminal 50. Therefore, the physical quantity can be acquired more easily.

Furthermore, according to the present embodiment, the deriving unit 11B derives the intensity of the exercise described above and thereafter according to whether or not the intensity indicated by the physical quantity acquired by the acquisition unit 11A is equal to or greater than a success threshold determined in advance as the lower limit value of the range considered to be successful. Therefore, the intensity of the self-weight exercise can be set more appropriately.

In the above embodiment, the case where the subject of the exercise support system 90 is a patient with type 2 diabetes has been described, but the present invention is not limited thereto. For example, a patient with metabolic syndrome may be applied as the subject, or a normal person who does not have any disease other than the patient may be applied as the subject.

In the above embodiment, the case where the technique of the present disclosure is realized by the exercise support system 90 including the exercise support device 10, the mobile terminal 40, and the wearable terminal 50 has been described, but the present invention is not limited thereto. For example, the technique of the present disclosure may be realized only by the mobile terminal 40 and the wearable terminal 50 without applying the exercise support device 10. As an exemplary embodiment in this case, an embodiment can be exemplified in which the mobile terminal 40 executes each process of the registration execution process, the exercise status registration process, and the intensity setting process according to the above embodiment and a process excluding communication with the mobile terminal 40. In this case, since the exercise support device 10 is unnecessary, the technique of the present disclosure can be realized at a lower cost as compared with the above embodiment.

Furthermore, the technique of the present disclosure may be realized only by the exercise support device 10 and the wearable terminal 50. As an exemplary embodiment in this case, it is possible to exemplify an embodiment in which the wearable terminal 50 executes each process of the registration request process and the exercise execution process according to the above embodiment and a process excluding communication with the wearable terminal 50. In this case, since the mobile terminal 40 is unnecessary, also in this case, the technique of the present disclosure can be realized at a lower cost as compared with the above embodiment.

In the above embodiment, the case where the default intensity (success threshold) of each self-weight exercise is common regardless of the type of the self-weight exercise has been described, but the present invention is not limited thereto. For example, different intensities may be applied as the default intensity for each type of self-weight exercise.

Furthermore, in the above-described embodiment, the case where the success threshold LI derived by the deriving unit 11B is registered in the intensity information database 13F as the lower limit value of the success range of the intensity of the subsequent self-weight exercise by the presentation unit 11C, so that the success threshold LI is indirectly displayed (presented) in the form of being incorporated in the exercise program by the display unit 45 of the mobile terminal 40 has been described, but the present invention is not limited thereto. For example, instead of the processing of Step 810 and Step 822 in the intensity setting processing (see Fig. 16), the success threshold LI may be directly displayed (presented) by transmitting the success threshold LI to the mobile terminal 40 of the corresponding processing subject and executing process of displaying the success threshold LI on the display unit 45 of the mobile terminal 40.

In the above embodiment, in the intensity setting process, the case where the success threshold LI is derived in the case where the self-weight exercise is a set exercise by a plurality of repetitive executions, has been described, but the present invention is not limited thereto. In a case where the self-weight exercise is only one exercise, the processing executed for one set in the intensity setting process may be executed for only the execution status information (hereinafter, referred to as "single execution status information") obtained according to the execution of the one self-weight exercise. Furthermore, in this case, the process of calculating the average value Av in Step 806 in the intensity setting process becomes unnecessary, and instead of the average value Av in the subsequent process, the intensity obtained from the single execution status information may be applied, and '1' may be applied as the predetermined threshold applied in the processing of Step 814.

Furthermore, in the embodiment described above, the case where the information including both the amount and the speed of the exercise in the three-dimensional direction is applied as the execution status information obtained from the wearable terminal 50 has been described, but the present invention is not limited thereto. For example, information including only one of the amount of exercise and the speed in the three-dimensional direction may be applied as the execution status information. In this case, the intensity of each self-weight exercise is derived using the applied physical quantity.

In addition, Formulas (1) to (2) applied in the above embodiment are examples, and it goes without saying that both the formulas may be changed as appropriate without departing from the spirit of the technique of the present disclosure.

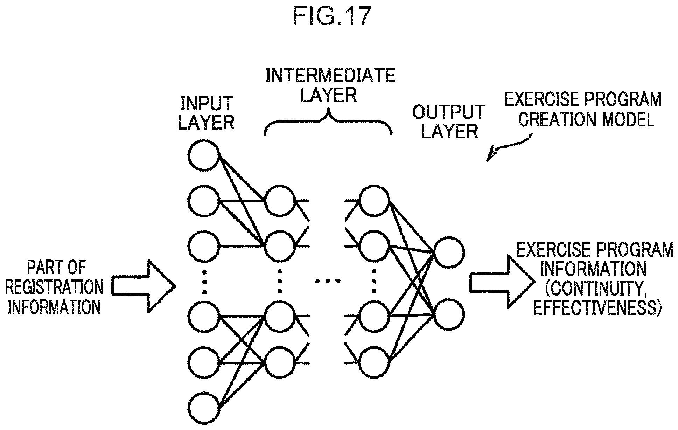

In the above embodiment, the case where the exercise program is prepared in advance for each gender and age of the subject has been described, but the present invention is not limited thereto. For example, as an example, an exercise program creation model that is a machine learning model for creating exercise program information suitable for the subject illustrated in Fig. 17 is created in advance. The exercise program information may be created for each subject using the exercise program creation model.

Note that the exercise program creation model illustrated in Fig. 17 is a neural network including an input layer, a plurality of intermediate layers, and an output layer. The gender and the age of the subject, which are a part of the registration information according to the above embodiment, are input to the input layer of the exercise program creation model. The output layer of the exercise program creation model outputs exercise program information suitable for the subject corresponding to the input information. The exercise program creation model is obtained in advance by causing a plurality of combinations of gender and age of an unspecified large number of subjects obtained in the past and exercise program information with high exercise continuity and effectiveness in the subjects to be learned as teacher data. In addition, as an application example of this mode, it may be a mode in which the physical function (physical strength, muscle strength, etc.) of the subject and the execution result of the first exercise are input to the input layer, and a plurality of combinations of the physical function and the execution result of the first exercise of an unspecified number of subjects obtained in the past and the exercise program information with high exercise continuity and effectiveness in the subjects are learned as teacher data, thereby constructing the exercise program creation model.

In addition, in the above embodiment, the case has been described in which whether or not the self-weight exercise is successful is determined by determining whether or not the intensity of the actually performed exercise is greater than or equal to the success threshold LI, but the present invention is not limited thereto. For example, an embodiment may be adopted in which a physical quantity indicating a form of the subject in a case where the subject performs the self-weight exercise using a wearable terminal or the like is acquired, and whether or not the self-weight exercise is successful is determined using the acquired physical quantity.

In this embodiment, when the physical quantity indicating the form is detected, for example, in a case where the self-weight exercise is push-up, a 3D motion sensor and a transmission device that wirelessly transmits a detection value by the 3D motion sensor are provided on the back or chest of the subject as an example. The physical quantity obtained by the 3D motion sensor is acquired via the transmission device. Then, in this case, it is possible to exemplify an embodiment of determining whether or not the push-up is successful by determining whether or not the acquired physical quantity is included in a range of the form that is preferable when the push-up is performed. Note that, in this embodiment, since the wearable terminal 50 according to the above embodiment is a wristband-type terminal, this cannot be applied. Instead of the wearable terminal 50, the 3D motion sensor and the transmission device may be directly provided by being attached to clothing, skin, or the like of the subject. However, even if the wearable terminal 50 is a wristband-type terminal, if a sensor portion is detachable from the band, the sensor portion may be attached to a belt having a size capable of being wound around the chest.

In the embodiment described above, the exercise program information is prepared for each subject attribute indicating the attribute (in the above embodiment, gender and age) of the subject, and the exercise program matching the subject to be applied is applied to the subject. However, the invention is not limited thereto. For example, only single exercise program information to be uniformly applied to all the subjects may be prepared, and the exercise program information may be applied to all the subjects. In this case, the load of the processing for selecting the exercise program information can be eliminated, and the storage capacity for the exercise program database 13D can be reduced.

Furthermore, in the above-described embodiment, a case of changing the event and the number of sets of the exercise to be performed for each of the three stages of 1 to 2 weeks (first period), 3 to 4 weeks (second period), and 5 to 12 weeks (third period) in the exercise program information has been exemplified, but the invention is not limited thereto. Conditions for changing the event and the number of sets of exercise in this case may be set as follows.

That is, the execution result of the exercise according to the exercise program information is evaluated for each exercise execution day of each week, and the degree of achievement is scored. Then, in a case where the score is a predetermined value or more, the process can proceed to the exercise program of a next week.

Furthermore, in the above-described embodiment, the case where a compensation for the provision of the exercise program information is imposed on the subject when the exercise program information is provided has been described, but the present invention is not limited thereto.

For example, a monthly fee for 1 month may be charged as an advance fee before the registration request process illustrated in Fig. 10 is executed, and the monthly fee may be automatically withdrawn from a designated account after 2 months from the start of exercise.

In addition, in the above embodiment, the case where the intensity in and after the second day, is set on the basis of the execution status of the last set of exercise on the previous day has been described, but the present invention is not limited thereto. For example, in a case where a plurality of sets of exercises is performed in a day, the intensity of the next exercise may be set in the day on the basis of the execution status of the previous set of exercises.

Furthermore, in the above embodiment, a case where a terminal including both an acceleration sensor and a gyro sensor is applied as the wearable terminal 50 has been described, but the present invention is not limited thereto. For example, a terminal including only one of the acceleration sensor and the gyro sensor may be applied as the wearable terminal 50.

Furthermore, in the above-described embodiment, for example, the following various processors can be used as a hardware structure of a processing unit that executes each process of the acquisition unit 11A, the deriving unit 11B, and the presentation unit 11C. As described above, the various processors include not only a CPU that is a general-purpose processor that functions as a processing unit by executing software (program), but also a dedicated electric circuit or the like that is a processor having a circuit configuration designed exclusively for executing specific processing such as a programmable logic device (PLD) that is a processor whose circuit configuration can be changed after manufacturing of a field-programmable gate array (FPGA) or an application specific integrated circuit (ASIC).

The processing unit may include one of these various processors, or may include a combination of two or more processors of the same type or different types (for example, a combination of a plurality of FPGAs or a combination of a CPU and an FPGA). In addition, the processing unit may be configured by one processor.

As an example in which the processing unit is configured by one processor, first, there is a mode in which one processor is configured by a combination of one or more CPUs and software as represented by a computer such as a client and a server, and this processor functions as a processing unit. Second, as represented by a system on chip (SoC) or the like, there is a form of using a processor that realizes a function of the entire system including a processing unit by one integrated circuit (IC) chip. As described above, the processing unit is configured using one or more of the above-described various processors as a hardware structure.

Furthermore, more specifically, an electric circuit (circuitry) obtained by combining circuit elements such as semiconductor elements can be used as a hardware structure of these various processors.

(Supplementary notes)

With regard to the above embodiments, the following supplementary notes are further disclosed.

(Supplementary note 1)

An exercise support device, comprising:

a memory;
at least one processor connected to the memory, wherein
the processor is further configured to:

acquire a physical quantity indicating an execution degree of an exercise by an exercise program indicating a predetermined self-weight exercise;
derive subsequent intensity of the exercise using the acquired physical quantity; and present the derived intensity.

(Supplementary note 2)

A non-transitory storage medium storing a program executable by a computer to perform an exercise support process, wherein
the exercise support process includes:

acquiring a physical quantity indicating an execution degree of an exercise according to an exercise program indicating a predetermined self-weight exercise;

deriving subsequent intensity of the exercise using the acquired physical quantity; and presenting the derived intensity.

The disclosure of Japanese Application No. 2019 -199835 is incorporated herein by reference in its entirety.

All documents, patent applications, and technical standards described in this specification are incorporated herein by reference to the same extent as if each individual document, patent application, and technical standard were specifically and individually described to be incorporated by reference.

**Claims**

1. An exercise support device comprising:

   an acquisition unit configured to acquire a physical quantity indicating an execution degree of an exercise according to an exercise program indicating a predetermined self-weight exercise;
   a deriving unit configured to derive subsequent intensity of the exercise using the physical quantity acquired by the acquisition unit; and
   a presentation unit configured to present the intensity derived by the deriving unit.

2. The exercise support device according to claim 1, wherein
   the exercise is defined by at least one of frequency, intensity, duration, or type.

3. The exercise support device according to claim 1 or 2, wherein
   the deriving unit derives the intensity using a physical quantity acquired immediately before by the acquisition unit.

4. The exercise support device according to claim 3, wherein
   the deriving unit derives the intensity by using the physical quantity acquired for at least one latest set of exercises by the acquisition unit as the physical quantity acquired immediately before, in a case in which the exercise is a set exercise by a plurality of repetitive executions.

5. The exercise support device according to any one of claims 1 to 4, wherein
   the deriving unit derives intensity of a next exercise.

6. The exercise support device according to claim 5, wherein
   the deriving unit derives intensity of a next set exercise as the intensity of the next exercise in a case in which the exercise is a set exercise by a plurality of repetitive executions.

7. The exercise support device according to any one of claims 1 to 6, wherein
the deriving unit derives the intensity as any one of a value increased from a previous value, a value decreased from the previous value, or a value same as the previous value.

8. The exercise support device according to any one of claims 1 to 7, wherein
the exercise is a set exercise by a plurality of repetitive executions.

9. The exercise support device according to any one of claims 1 to 8, wherein
the acquisition unit acquires the physical quantity as a value indicating at least one of an exercise amount or a speed in a three-dimensional direction when the exercise is executed.

10. The exercise support device according to any one of claims 1 to 9, wherein
the acquisition unit acquires the physical quantity using a wearable terminal.

11. The exercise support device according to any one of claims 1 to 10, wherein
the deriving unit derives intensity of a subsequent exercise according to whether or not the intensity indicated by the physical quantity acquired by the acquisition unit is greater than or equal to a success threshold set in advance as a lower limit value of a range in which the exercise is considered to be successful.

12. An exercise support system comprising:

the exercise support device according to any one of claims 1 to 11; and
a terminal including a transmission unit that transmits information indicating the physical quantity to the acquisition unit of the exercise support device.

13. An exercise support method comprising:

acquiring a physical quantity indicating an execution degree of an exercise according to an exercise program indicating a predetermined self-weight exercise;
deriving subsequent intensity of the exercise using the acquired physical quantity; and
presenting the derived intensity.

14. A program for causing a computer to execute a process of:

acquiring a physical quantity indicating an execution degree of an exercise according to an exercise program indicating a predetermined self-weight exercise;
deriving subsequent intensity of the exercise using the acquired physical quantity; and
presenting the derived intensity.

# FIG.1

90 EXERCISE SUPPORT SYSTEM

OPERATING COMPANY
(PHARMACEUTICAL COMPANY) 10

SUBJECT 40

## MOBILE TERMINAL

### EXERCISE SUPPORT DEVICE

41 CPU

42 MEMORY

44 INPUT UNIT

B2

STORAGE UNIT 43

REGISTRATION REQUEST PROGRAM 43A

EXERCISE EXECUTION PROGRAM 43B

DISPLAY UNIT 45

WIRELESS COMMUNICATION UNIT 48

11 CPU

12 MEMORY

14 INPUT UNIT

15 DISPLAY UNIT

18 COMMUNI-CATION I/F UNIT

16 R/W    17 ◎

13

STORAGE UNIT

REGISTRATION EXECUTION PROGRAM 13A

EXERCISE STATUS REGISTRATION PROGRAM 13B

INTENSITY SETTING PROGRAM 13C

EXERCISE PROGRAM DATABASE 13D

EXERCISE HISTORY INFORMATION DATABASE 13E

INTENSITY INFORMATION DATABASE 13F

B1

80 NETWORK

50 WEARABLE TERMINAL

51 3D MOTION CENSOR

EP 4 053 848 A1

## FIG.2

SUBJECT

OPERATING COMPANY
(PHARMACEUTICAL COMPANY)

WEARABLE TERMINAL — 50

MOBILE TERMINAL — 40

TRANSMISSION UNIT — 41A

DISPLAY UNIT — 45

EXECUTION STATUS INFORMATION

INTENSITY

EXERCISE SUPPORT DEVICE — 10

ACQUISITION UNIT — 11A

DERIVING UNIT — 11B

PRESENTATION UNIT — 11C

## FIG.3

SUBJECT

50

UPPER ARM

## FIG.4

SUBJECT

LOWER THIGH

50

## FIG.5

VERTICAL DIRECTION

UPPER ARM

50

ELBOW

52

SUBJECT

# FIG.6

SUBJECT

50

LOWER THIGH

52

HORIZONTAL DIRECTION

WAIST

# FIG.7

EXERCISE PROGRAM DATABASE

| SUBJECT ATTRIBUTE | | EXERCISE TYPE | SPECIFIED TYPE | PERIOD | | |
|---|---|---|---|---|---|---|
| GENDER | AGE | | | 1 TO 2 WEEKS | 3 TO 4 WEEKS | 5 TO 12 WEEKS |
| MALE | 20 ~ 29 | RESISTANCE EXERCISE (SELF-WEIGHT EXERCISE) | INTENSITY RANGE (DEGREE) | B1~B2 | B3~B4 | B5~B |
| | | | EXECUTION TARGET | X1 EVENT,Y1SET | X2 EVENT,Y2SET | X3 EVENT,Y3SET |
| | | | EXERCISE TYPE | PUSH-UP, LEG-RAISE ・・・ | | |
| | 30 ~ 49 | ・ | ・ | ・ | ・ | ・ |
| | ・ | ・ | ・ | ・ | ・ | ・ |
| FEMALE | ・ | ・ | ・ | ・ | ・ | ・ |

## FIG.8

EXERCISE HISTORY INFORMATION DATABASE

| SUBJECT ID | SUBJECT INFORMATION | EXERCISE TYPE | EXERCISE HISTORY | | |
|---|---|---|---|---|---|
| | | | 1 TO 2 WEEKS | 3 TO 4 WEEKS | 5 TO 12 WEEKS |
| K001 | (NAME, GENDER, AGE) | PUSH-UP | (EXECUTION STATUS INFORMATION) | (EXECUTION STATUS INFORMATION) | (EXECUTION STATUS INFORMATION) |
| | | LEG-RAISE | (EXECUTION STATUS INFORMATION) | (EXECUTION STATUS INFORMATION) | (EXECUTION STATUS INFORMATION) |
| | | ⋮ | (EXECUTION STATUS INFORMATION) | (EXECUTION STATUS INFORMATION) | (EXECUTION STATUS INFORMATION) |
| | | | (EXECUTION STATUS INFORMATION) | (EXECUTION STATUS INFORMATION) | (EXECUTION STATUS INFORMATION) |
| K002 | ⋮ | ⋮ | ⋮ | ⋮ | ⋮ |
| ⋮ | ⋮ | ⋮ | ⋮ | ⋮ | ⋮ |

## FIG.9

INTENSITY INFORMATION DATABASE

| SUBJECT ID | EXERCISE TYPE | INTENSITY (DEGREE) | | | |
|---|---|---|---|---|---|
| | | FIRST DAY | SECOND DAY | THIRD DAY | · · · |
| K001 | PUSH-UP | a1 | a2 | a3 | · · · |
| | LEG-RAISE | b1 | b2 | b3 | · · · |
| | · · · | · · · | · · · | · · · | · · · |
| K002 | · · · | · · · | · · · | · · · | · · · |
| · · · | · · · | · · · | · · · | · · · | · · · |

## FIG.10

REGISTRATION REQUEST PROCESS

REGISTRATION SCREEN DISPLAY ~100

PREDETERMINED INFORMATION IS INPUT? — 102

N

Y

SEND REGISTRATION INFORMATION ~104

EXERCISE PROGRAM INFORMATION IS RECEIVED? — 106

N

Y

STORE EXERCISE PROGRAM INFORMATION — 108

COMPENSATION PAYMENT PROCESS ~110

END

# FIG.11

REGISTRATION INFORMATION 45

PLEASE INPUT INFORMATION ABOUT YOU

NAME

GENDER

AGE

END 45A

# FIG.12

REGISTRATION EXECUTION PROCESS

READ EXERCISE PROGRAM INFORMATION CORRESPONDING TO REGISTRATION INFORMATION ～200

SEND EXERCISE PROGRAM INFORMATION TO TERMINAL OF TRANSMISSION SOURCE ～202

REGISTER SUBJECT ID, NAME, GENDER, EXERCISE TYPE IN EACH DATABASE ～204

COMPENSATION PAYMENT INSTRUCTION PROCESS ～206

END

# FIG.13

```
┌─────────────────────────────────────┐
│     EXERCISE EXECUTION PROCESS        │
└─────────────────────────────────────┘
                  │
┌─────────────────────────────────────┐
│  READ EXERCISE PROGRAM INFORMATION   │ 600
└─────────────────────────────────────┘
                  │
            ╱──────────╲          602
          ╱   FIRST DAY   ╲  N
          ╲  OF EXERCISE  ╱───────────┐
            ╲──────────╱              ▼
                │ Y          ┌──────────────────┐
                │            │  READ SUCCESS     │ 604
                │            │   THRESHOLD       │
                │            └──────────────────┘
                │                     │
                │            ┌──────────────────┐
                │            │  SET AS EXERCISE  │ 606
                │            │    PROGRAM        │
                │            └──────────────────┘
                │◄────────────────────┘
                │
┌─────────────────────────────────────┐
│   DISPLAY EXERCISE PROGRAM           │ 608
│       DISPLAY SCREEN                 │
└─────────────────────────────────────┘
                │
┌─────────────────────────────────────┐
│  START RECEIVING AND STORING         │ 610
│  EXECUTION STATUS INFORMATION        │
└─────────────────────────────────────┘
                │
          ╱──────────╲        612
   N    ╱ PREDETERMINED ╲
◄──────╲  TIME ELAPSED? ╱
        ╲──────────╱
              │ Y
┌─────────────────────────────────────┐
│  READ EXECUTION STATUS INFORMATION   │ 614
└─────────────────────────────────────┘
                │
┌─────────────────────────────────────┐
│  SEND EXECUTION STATUS INFORMATION   │ 616
└─────────────────────────────────────┘
                │
          ╱──────────╲        618
        ╱  END TIMING  ╲  N
        ╲  HAS COME?   ╱──────
          ╲──────────╱
              │ Y
┌─────────────────────────────────────┐
│  START RECEIVING AND STORING         │ 620
│  EXECUTION STATUS INFORMATION        │
└─────────────────────────────────────┘
                │
        ┌──────────────┐
        │     END       │
        └──────────────┘
```

## FIG.14

EXERCISE PROGRAM DISPLAY SCREEN　　　　45

EXERCISE PROGRAM FOR ●●● IS SHOWN AS FOLLOWS

| SPECIFIED TYPE | PERIOD | | |
|---|---|---|---|
| | 1 TO 2 WEEKS | 3 TO 4 WEEKS | 5 TO 12 WEEKS |
| INTENSITY RANGE (DEGREE) | B1～B2 | B3～B4 | B5～B6 |
| EXECUTION TARGET | X1 EVENT, Y1 SET | X2 EVENT, Y2 SET | X3 EVENT, Y3 SET |
| EXERCISE TYPE | PUSH-UP, LEG-RAISE ・・・ | | |

END　　45A

# FIG.15

EXERCISE STATUS REGISTRATION PROCESS

STORE RECEIVED EXECUTION STATUS
INFORMATION IN EXERCISE HISTORY
INFORMATION DATABASE ⟶ 700

END TIMING
HAS COME? — 702 N

Y

END

# FIG.16

INTENSITY SETTING PROCESS

READ EXECUTION INFORMATION OF ALL EVENTS FOR SUBJECT ON PREVIOUS DAY ～800

EXTRACT INFORMATION OF LAST ONE SET ～802

CONVERT EXECUTION STATUS INFORMATION INTO INTENSITY ～804

CALCULATE AVERAGE VALUE AV OF INTENSITY ～806

808
SECOND DAY SINCE START OF EXERCISING?

N

Y
810
REGISTER Av AS SUCCESS THRESHOLD LI

DERIVE NUMBER OF SUCCESS N ～812

814
N ≥ PREDETERMINED THRESHOLD?

N

Y
816
$\alpha = (HI-LI)/\beta$

818
$\alpha = 0$

$LI = Av + \alpha$ ～820

REGISTER LI AS NEXT SUCCESS THRESHOLD LI ～822

824
DETERMINE WHETHER TO END PROCESSING ON ANY OTHER REMAINING EVENT

N

Y

END

# FIG.17

INPUT LAYER

INTERMEDIATE LAYER

OUTPUT LAYER

EXERCISE PROGRAM CREATION MODEL

PART OF REGISTRATION INFORMATION

EXERCISE PROGRAM INFORMATION (CONTINUITY, EFFECTIVENESS)

**INTERNATIONAL SEARCH REPORT**

| International application No. |
|---|
| PCT/JP2020/039364 |

A.  CLASSIFICATION OF SUBJECT MATTER
G16H 20/30(2018.01)i; G06Q 50/10(2012.01)i
FI: G16H20/30; G06Q50/10

According to International Patent Classification (IPC) or to both national classification and IPC

B.  FIELDS SEARCHED

Minimum documentation searched (classification system followed by classification symbols)
G16H10/00-80/00; G06Q10/00-99/00

Documentation searched other than minimum documentation to the extent that such documents are included in the fields searched
Published examined utility model applications of Japan       1922–1996
Published unexamined utility model applications of Japan     1971–2020
Registered utility model specifications of Japan             1996–2020
Published registered utility model applications of Japan     1994–2020

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X | JP 2018-201905 A (FUJI XEROX CO., LTD.) 27 December 2018 (2018-12-27) paragraphs [0005]-[0162], fig. 1-3 | 1-14 |
| A | WO 2016/092912 A1 (SONY CORP.) 16 June 2016 (2016-06-16) entire text, all drawings | 1-14 |
| A | JP 2011-520556 A (QUALCOMM INCORPORATED) 21 July 2011 (2011-07-21) entire text, all drawings | 1-14 |
| A | JP 2006-263002 A (NIPPON TELEGRAPH AND TELEPHONE CORP.) 05 October 2006 (2006-10-05) entire text, all drawings | 1-14 |
| A | JP 2010-82054 A (BROTHER INDUSTRIES, LTD.) 15 April 2010 (2010-04-15) entire text, all drawings | 1-14 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 December 2020 (24.12.2020) | 12 January 2021 (12.01.2021) |

| Name and mailing address of the ISA/ Japan Patent Office 3-4-3, Kasumigaseki, Chiyoda-ku, Tokyo 100-8915, Japan | Authorized officer Telephone No. |
|---|---|

Form PCT/ISA/210 (second sheet) (January 2015)

**INTERNATIONAL SEARCH REPORT**
Information on patent family members

International application No.

PCT/JP2020/039364

| Patent Documents referred in the Report | Publication Date | Patent Family | Publication Date |
|---|---|---|---|
| JP 2018-201905 A | 27 Dec. 2018 | US 2018/0345082 A1 paragraphs [0012]-[0130], fig. 1-3 | |
| WO 2016/092912 A1 | 16 Jun. 2016 | US 2017/0266492 A1 entire text, all drawings EP 3231484 A1 | |
| JP 2011-520556 A | 21 Jul. 2011 | US 2009/0292178 A1 entire text, all drawings WO 2009/142932 A1 KR 10-2011-0010644 A CN 102036718 A | |
| JP 2006-263002 A | 05 Oct. 2006 | (Family: none) | |
| JP 2010-82054 A | 15 Apr. 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009142333 A
- JP 2010252985 A
- JP 2016507114 A
- JP 2019199835 A

**Non-patent literature cited in the description**

- *Japan Journal of Physical Education, Health and Sport Sciences,* 2012, vol. 57, 191-199